# EUROPEAN PATENT APPLICATION

(11) **EP 0 677 587 A1**
(43) Date of publication of application: **18.10.1995**
(21) Application number: 95105481.6
(22) Date of filing: 12.04.1995
(51) Int. Cl.: C12P 19/12

(54) **Processes for the production of trehalose**

(30) Priority: 15.04.1994 JP 102319/94; 02.12.1994 JP 329387/94
(71) Applicant: KUREHA CHEMICAL INDUSTRY CO., LTD., Chuo-ku Tokyo (JP)
(72) Inventor: Takahashi, Eisaku, Nerima-ku, Tokyo (JP); Kase, Toshiya, Warabi-shi, Saitama (JP); Konai, Yutaka, Machida-shi, Tokyo (JP)
(74) Representative: Boeters, Hans Dietrich, Dr.

(57) **Abstract**

This invention relates to processes for the production of trehalose using sucrose as a sole raw material. The characteristic feature of the present invention is a production of trehalose by a reaction of sucrose, sucrose phosphorylase, glucose isomerase and trehalose phosphorylase in the presence of an inorganic phosphoric acid and/or a salt thereof followed by isolation of trehalose.

## Description

### Field of the Invention

This invention relates to effective and industrial processes for the production of trehalose from sucrose. The resultant trehalose can be used for the production of sweeteners, medicines and cosmetics.

### Background of the Invention

Recently, various oligosaccharides have focused the spotlight of attention and their developments are now under progress. Trehalose is a disaccharide widely distributing in microorganisms, animals and plants and its applications such as a sweetener (Japanese Published Unexamined Patent Application No. 240758 (1988)) and a protectant against drying of protein (Publication of the Translation of International Patent Application No. 500562 (1988)) have been proposed.

Heretofore, production of trehalose such as by extraction from the above mentioned natural sources and fermentation of microorganisms, for example, yeasts, Arthrobacter, Nocardia and Corynebacterium have been known. However, these procedures could not provide high productivity and required troublesome procedures for the isolation and purification of the aimed product and, therefore, are unsuitable for the industrial production of trehalose.

An enzymic preparation of trehalose with maltose phosphorylase and trehalose phosphorylase from maltose via β-glucose 1-phosphate is known (Japanese Published Examined Patent Application No. 60998 (1988)). However, cost of raw material maltose, and supply and cost of maltose phosphorylase have not been satisfactory. Trehalose phosphorylase which produce trehalose from β-glucose 1-phosphate and glucose is not sufficiently stable and, furthermore, culture of Euglena gracilis for the preparation of the enzyme is not always easy.

While, a trehalose phosphorylase obtained from Flammulina velutipes is known to produces trehalose from α-glucose 1-phosphate and glucose at about pH 7 (FEMS Microbiology Letters, 55, 147-150 (1988)). However, the trehalose phosphorylase obtained from Flammulina velutipes is unstable, difficult to be purified and can not be used for a long period of time at elevated temperature, thus, unsuitable for the industrial production of trehalose.

The present inventors have been investigating a process for the production of trehalose from an easily and industrially available source of sucrose, and found a trehalose phosphorylase to produce trehalose from glucose and α-glucose 1-phosphate which are easily prepared from sucrose (Japanese Patent Application No. 76461 (1994)). In addition, the inventors found a process for the production of α-glucose 1-phosphate by a reaction of sucrose with an inorganic phosphoric acid and/or a salt thereof catalyzed by sucrose phosphorylase, and then established a process for the production of trehalose by a reaction of the produced α-glucose 1-phosphate with glucose catalyzed by said trehalose phosphorylase, and filed a patent application (Japanese Patent Application No. 76393 (1994)).

However, the above mentioned process for the production of trehalose established by the present inventors required glucose in addition to sucrose as a raw material sugar.

The inventors further investigated the production of trehalose from sucrose as a sole raw material without glucose and accomplished the processes of the present invention. After eager investigation to solve the above problems, the inventors found that a reaction of sucrose with sucrose phosphorylase, trehalose phosphorylase and glucose isomerase in the presence of an inorganic phosphoric acid and/or a salt thereof produced trehalose and accomplished the present invention.

### Summary of the Invention

One object of the present invention is to provide a process for the production of trehalose by a reaction of sucrose with an inorganic phosphoric acid and/or a salt thereof, catalyzed by sucrose phosphorylase, glucose isomerase and trehalose phosphorylase to prepare trehalose followed by isolation of trehalose.

The other object of the present invention is to provide a series of processes for the production and isolation of trehalose by carrying out the following reactions serially: (1) a process for the production of α-glucose 1-phosphate and fructose by a reaction of sucrose with an inorganic phosphoric acid and/or a salt thereof catalyzed by sucrose phosphorylase; (2) a process for the production of glucose by a reaction of fructose produced by the preceding process (1) with glucose isomerase; (3) a process for the production of trehalose and regeneration of inorganic phosphoric acid by a reaction of α-glucose 1-phosphate produced by the preceding process (1) with glucose produced by the preceding process (2) catalyzed by trehalose phosphorylase.

A further object of the present invention is to provide a series of processes for the production of trehalose by performing the following reactions serially: (process 1) a reaction of sucrose with an inorganic phosphoric acid and/or a salt catalyzed by sucrose phosphorylase; (process 2) addition of glucose isomerase after an appropriate reaction time to the mixture of α-glucose 1-phosphate and fructose without separation to start the preparation of glucose from fructose; and (process 3) a reaction of α-glucose 1-phosphate produced by the (process 1) and glucose produced by the (process 2) after an appropriate reaction time to convert serially into trehalose catalyzed by trehalose phosphorylase, followed by an isolation of produced trehalose.

### Detailed Description of the Invention and Preferred Embodiment

One of the characteristic features of the present invention is that three kinds of enzymes used in the invention do not disturb their functions with each other, nor are inhibited by their substrates or products each other. This characteristic feature led to a series of reactions for the formation of α-glucose 1-phosphate and fructose from sucrose catalyzed by sucrose phosphorylase, then additions of glucose isomerase and trehalose phosphorylase after a while to cause the following reaction of α-glucose 1-phosphate which was already produced by the preceding reaction and α-glucose 1-phosphate prepared continuously from unaltered sucrose, with glucose produced from fructose with glucose isomerase to produce trehalose as the object of the present invention. Furthermore, inorganic phosphoric acid is produced in the above reaction and can be recycled for the reaction with sucrose, thus the required amount of inorganic phosphoric acid and/or a salt thereof can be reduced far from that of stoichiometric amount. This is an advantage from the viewpoint of practical production.

In the present invention, sucrose, an inorganic phosphoric acid and/or a salt thereof, sucrose phosphorylase, trehalose phosphorylase, and glucose isomerase may be mixed and reacted for the production of trehalose. Alternatively, these reagents can be used in stepwise reactions starting from sucrose, followed by serial addition of an inorganic phosphoric acid and/or a salt thereof, sucrose phosphorylase, glucose isomerase and trehalose phosphorylase for the production of trehalose.

In the process of the present invention, a reaction may be initiated by mixing sucrose and three kinds of enzymes which is a preferred embodiment of the invention. Because, immediately after the start of formation of α-glucose 1-phosphate and fructose by a reaction of sucrose and sucrose phosphorylase, formation of glucose from fructose with glucose isomerase begins. Then, the formation of trehalose by the action of trehalose phosphorylase simultaneously starts with the formation of glucose providing a maximum possibility for the conversion of intermediates α-glucose 1-phosphate and fructose into trehalose. Thus, the possible losses of α-glucose 1-phosphate and fructose by side reactions will become minimum. Furthermore, continuous consumption of glucose provides a maximum reaction rate of conversion of fructose into glucose with glucose isomerase.

Furthermore, to start a series of reactions by mixing sucrose and three kinds of enzymes makes the recycle and reuse of an inorganic phosphoric acid and/or a salt thereof possible and minimizes their consumption which leads to the lowest inhibition of trehalose phosphorylase activity and results in the maximum production of trehalose with trehalose phosphorylase.

In the present invention, a sucrose phosphorylase means an enzyme which catalyzes the production of α-glucose 1-phosphate and fructose from sucrose and an inorganic phosphoric acid and/or a salt thereof. Trehalose phosphorylase means an enzyme which catalyzes the production of trehalose from α-glucose 1-phosphate and glucose. In addition, glucose isomerase means an enzyme which catalyzes the formation of glucose from fructose.

Sucrose phosphorylase is a known enzyme and any enzyme which catalyzes the reaction of sucrose with an inorganic phosphoric acid and/or a salt thereof to yield α-glucose 1-phosphate and fructose can be used regardless of their sources. The enzymes derived from the cultivation of microorganisms can be used as well as commercial products. For example, sucrose phosphorylases produced by microorganisms such as Leuconostoc mesenteroides and Pseudomonas saccharophila can be used.

Any glucose isomerase which catalyzes the preparation of glucose from fructose and any enzyme having such function can be used regardless of their sources. The enzyme derived from the cultivation of microorganisms can be used as well as commercial products. For example, glucose isomerase produced by microorganisms such as Streptomyces and Arthrobacter can be used.

Any stable trehalose phosphorylase which catalyzes the reaction of α-glucose 1-phosphate with glucose to give trehalose can be used regardless of their sources. For example, enzymes derived from Grifola, Pleurotus, Lyophyllum, Lentinus, Agaricus, Trametes, Coriolus, Lenzites and Schizophyllum can be used. Particularly, the trehalose phosphorylase with excellent thermal stability discovered by the present inventors is preferably used (Japanese Patent Application No. 76461 (1994)).

Thus, sucrose phosphorylase, glucose isomerase and trehalose phosphorylase used in the present invention can be obtained from microorganisms and their variant strains producing these enzymes.

The variants of these enzyme producing microorganisms can be obtained by treatment of the above mentioned enzyme producing microorganisms with conventional variant inducers such as N-methyl-N'-nitro-N-nitrosoguanidine (NTG), ethyl methanesulfonate (EMS) or ultraviolet ray.

Sucrose phosphorylase, glucose isomerase and trehalose phosphorylase used in the present invention can be obtained by the cultivation of recombinant host microorganisms which were inserted with a gene coding the enzyme or the modified enzyme thereof.

Furthermore, the enzymes used for the present invention can be used both in crude and purified forms or in immobilized forms prepared by conventional methods such as binding with a carrier, crosslinking, inclusion in a gel and micro-encapsulation. Additionally, living microorganisms can be used as immobilized bio-catalysts prepared by an inclusion and immobilization method using polyacrylamides, κ -carrageenan, alginic acid, photo-crosslinking resin prepolymer.

The required amount of these catalysts is not restrictive and the optimal quantity may be cost-effectively determined. Generally 0.01 active unit or over, preferably 0.1-100 units for 1 g of sucrose determined by the method disclosed later in the examples is used.

The concentrations of raw material sucrose for carrying out the present invention is 0.01M or over, preferably 0.05-2M.

The inorganic phosphoric acid and/or a salt thereof include conventional inorganic phosphoric acid and a salt thereof, for example, orthophosphoric acid, sodium phosphate, potassium phosphate, sodium dihydrogenphosphate and potassium dihydrogenphosphate may be used, preferably in a form of phosphate buffer solution.

In the present invention, 0.01 millimole to one mole, preferably 0.02 millimole to 0.5 mole of the inorganic phosphoric acid and/or a salt thereof to one mole of raw material sugar is used.

The reactions in the present invention are carried out at pH 2-10, preferably pH 4-9 and temperatures of 10-80°C, preferably 15-60°C.

The present invention is performed under reduced, normal or elevated pressure, and 0.01-1 kg/cm² (absolute pressure) for reduced pressure and 1-10 kg/cm² (absolute pressure) for elevated pressure are used.

The reaction of the present invention may be performed in the presence of air, however, in the inert gas such as nitrogen or argon, or under anaerobic atmosphere in the absence of oxygen is preferable for the improved yield of trehalose. The reaction under anaerobic atmosphere provides smooth enzymic reaction even with un-purified glucose isomerase and gives similar yield of trehalose with that of purified glucose isomerase.

After the completion of formation of trehalose, the enzyme is inactivated, if necessary, and the insoluble matters are removed from the reaction mixture by centrifugation or filtration to give a clear solution containing sugar compositions. The sugar compositions may be used as it is or trehalose in the composition may be used after isolation and purification, if necessary. For example, adsorption of trehalose to active charcoal for the removal of glucose and fructose followed by elution with 20% aqueous ethanol may be carried out to give a sugar solution mainly containing trehalose.

After the completion of formation of trehalose, the enzyme in the reaction mixture is inactivated, if necessary, and the reaction mixture is treated with an invertase to remove unaltered sucrose and the enzyme is inactivated if necessary. The insoluble matters are removed from the reaction mixture by centrifugation or filtration to give a clear sugar composition mainly containing trehalose.

Any invertase which forms fructose and glucose by hydrolysis of sucrose may be used regardless of their sources. Both commercial products and cultured products from microorganisms producing these enzymes can be used. For example, invertases produced by Saccharomyces cerevisiae and Candida utilis can be used.

Hydrolysis of sucrose with an invertase is carried out at pH 2-10, preferably pH 4-9 and at temperatures of 10-80°C, preferably 15-60°C . The amount of invertase used for the hydrolysis is not restrictive and the optimal requirement is determined from the viewpoint of cost-effectiveness. Generally, 0.01 unit or over, preferably 0.1-100 units of invertase is used for 1 g of sucrose. One unit of invertase is defined as an amount required to decompose 1 µ mole of sucrose at 20°C and pH 4.0 for 1 minute.

The sugar compositions obtained by the invertase treatment can be used as it is according to the purpose of uses or may be isolated and purified, if necessary. For example, adsorption of the composition to active charcoal for the removal of glucose and fructose followed by elution with 20% aqueous ethanol gives a sugar solution mainly containing trehalose.

As described above, combinations of three kinds of enzymic reactions in the presence of an inorganic phosphoric acid and/or a salt thereof easily convert sucrose into trehalose according to the present invention.

The present invention will be practically explained by the following Preparation examples and Examples, however, the scope of the present invention is not restricted by these examples.

### Preparation example 1

### (Preparation of trehalose phosphorylase)

In 600 ml of 20 mM Tris-HCl buffer (containing 1 mM EDTA and 1 mM dithiothreitol, pH 7.5, hereinafter the same composition will be used), 295 g of fresh fruit bodies of commercial Grifola frondosa was placed and disrupted with Waring Blendor™ (Waring Products Corp., a division of Dynamics Corp. of America). The mixture was centrifuged and 780 ml of a supernatant of crude enzyme solution was isolated. In the crude enzyme solution, ammonium sulfate was added up to 60% saturation and the precipitate was collected by centrifugation. The obtained precipitate was dissolved in 20 mM Tris-HCl buffer containing 30% ammonium sulfate and charged in a column (15 mm diameter x 140 mm length) of 24 ml of Butyl-Toyopearl™ 650 (TOSOH Corp.) equilibrated with the same buffer. The column was washed with the same buffer and eluted with 250 ml linear gradient of 0-30% ammonium sulfate in 20 mM Tris-HCl buffer. The active enzyme fraction was dialyzed against 20 mM citric acid-sodium hydroxide buffer (containing 1 mM EDTA and 1 mM dithiothreitol , pH 6.0, hereinafter the same buffer composition will be used). The eluate was charged in a column (15 mm diameter x 110 mm length) of 20 ml of AF-Blue Toyopearl™ 650 (TOSOH Corp.) equilibrated with the same buffer. The column was washed with the same buffer and eluted with 200 ml linear gradient of 0-0.5M potassium chloride in 20 mM citrate-sodium hydroxide buffer. The active enzymic fraction was concentrated with a hollow fiber ultrafiltration apparatus to give 3.1 ml of the enzyme solution. The resultant enzyme solution showed trehalose phosphorylase activity and specific activity of 10.8 unit/ml and 3.48 unit/mg protein, respectively.

The enzyme activity was determined by the following method. That is, 10.8 g of trehalose, 860 mg of glutathione and 17.2 mg of EDTA 2Na were dissolved in 57 mM potassium phosphate buffer (pH 7.0), and made 100 ml in total. In 1,400 µl of the solution, 100 µl each of 20 mM NADP⁺ aqueous solution, 26 mM magnesium chloride aqueous solution, 1.34 mM glucose 1,6-diphosphate aqueous solution, 31 unit/ml of phosphoglucomutase aqueous solution, 35 unit/ml of glucose-6-phosphate dehydrogenase and a test solution were mixed and incubated at 30°C. The formed NADPH was determined with the passage of time with the absorbance at 340 nm wave length. One unit of enzyme was defined as an amount required to form 1 µmole of NADPH under these conditions for 1 minute.

### Preparation example 2

### (Preparation of sucrose phosphorylase)

In 20 ml of 20 mM HEPES buffer (containing 1 mM EDTA and 1 mM dithiothreitol, pH 7.0), 5 mg of a commercial sucrose phosphorylase derived from Leuconostoc mesenteroides (Kikkoman Corp.) was dissolved to give a solution with activity of 18 unit/ml.

The enzyme activity was determined by the following method. That is, 9.78 g of sucrose, 860 mg of glutathione and 17.2 mg of EDTA 2Na were dissolved in 57 mM potassium phosphate buffer (pH 7.0), and made 100 ml in total. In 1,400 µl of the solution, 100 µl each of 20 mM NADP⁺ aqueous solution, 26 mM magnesium chloride aqueous solution, 1.34 mM glucose 1,6-diphosphate aqueous solution, 31 unit/ml of phosphoglucomutase aqueous solution, 35 unit/ml of glucose-6-phosphate dehydrogenase aqueous solution and a test enzyme solution were mixed and incubated at 30°C. The amount of formed NADPH was determined with the passage of time with the absorbance at 340 nm wave length. One unit of the enzyme was defined as an amount required to form 1 µmole of NADPH under the condition in a minute.

### Preparation example 3

### (Preparation of glucose isomerase)

In 85 ml of 20 mM HEPES buffer (containing 1 mM EDTA and 1 mM dithiothreitol, pH 7.0), 16.5 g of a commercial crude glucose isomerase derived from Streptomyces (Nagase Biochemicals Ltd.) was suspended and incubated with 160 mg of lysozyme (Sigma Chemical Co.) at 35°C for six hrs. and centrifuged to give 140 ml of supernatant. The supernatant was added with ammonium sulfate to give 30% saturation, allowed to stand and centrifuged to give 167 ml of supernatant. In 167 ml of the supernatant, ammonium sulfate was added up to 60% saturation to give precipitate. The precipitate was collected and dissolved in water and dialyzed against water to give 13.3 ml of enzyme solution. The enzyme solution was referred to 'ammonium sulfate precipitated glucose isomerase aqueous solution'. The glucose isomerase activity of the enzyme solution was 3.8 unit/ml. The enzyme solution was further purified with Super Q Toyopearl (TOSOH Corp.) chromatography and gel permeation chromatography(GPC) using TSKgel 3000SW (TOSOH Corp.) to give an enzyme solution having enzyme activity of 16.2 unit/ml and specific activity of 0.5 unit/mg protein, respectively. This enzyme solution was referred to 'purified glucose isomerase aqueous solution'.

The enzyme activity was determined by the following method. That is, 100 µl each of 0.5M fructose aqueous solution, 0.5M HEPES buffer (pH 7.0), 0.1M magnesium sulfate aqueous solution, purified water and a test enzyme solution were mixed and incubated at 35°C for 12 min. The amount of the formed glucose was determined with a glucose determination kit (Glucose CII-test Wako, Wako Pure Chemical Ltd.). One unit of the enzyme was defined as an amount required to form 1 µmole of glucose under these conditions for 1 minute.

### [Example 1]

In test tubes each having a screw cap, 0.9 ml of 1M sucrose aqueous solution, 0.12 ml of 500 mM potassium phosphate buffer (pH 7.0), 0.4 ml of 750 mM HEPES buffer (pH 7.0), 0.06 ml of 1M magnesium sulfate aqueous solution, 0.29 ml of 10.8 unit/ml of trehalose phosphorylase aqueous solution prepared by Preparation example 1, 0.18 ml of 18 unit/ml of sucrose phosphorylase aqueous solution prepared by Preparation example 2, 0.19 ml of 16.2 unit/ml of purified glucose isomerase aqueous solution prepared by Preparation example 3 and 0.86 ml of purified water were placed and mixed. The air in the test tubes was replaced with argon gas and the reaction mixture was incubated at 30°C for 0, 24, 48, 72 and 96 hrs., respectively. At the end of each reaction time, the reaction mixture was heated 100°C for 10 min. to inactivate the enzymes. The amount of sucrose and trehalose in the reaction mixture were determined by the following method. The concentration of sucrose was 290, 156, 39, 2.7 and 2.3 mM, respectively, and that of trehalose was 0, 111, 219, 255 and 260 mM, respectively. The yield of trehalose from sucrose was 90 mole%.

The quantitation of trehalose and sucrose was carried out with a high performance liquid chromatography using a polyamine column (YMC Pack™, Polyamine II, 4.6 mm diameter and 250 mm length, YMC Co., Ltd.), eluting solution of a mixture of acetonitrile: water = 70:30, flow rate of 1 ml/min., column temperature of 35°C , and a differential refractometer with cell temperature of 35°C . The retention time of sucrose and trehalose was 12.5 and 15.7 min., respectively.

### [Example 2]

In 2.8 ml of the resultant reaction mixture obtained by Example 1, 100 µl of an invertase aqueous solution (390 unit/ml, Wako Pure Chemical Ind. Ltd.) was added and incubated at 50°C for 60 min., then the enzyme was inactivated by heating at 100°C for 10 min. The treated sugar solution was analyzed by the method described in Example 1 and 251 mM of trehalose was found, but no sucrose was detected. Then, the treated solution was charged to a charcoal (Wako Pure Chemical Ind. Ltd.) column (25 mm diameter and 100 mm length) equilibrated with purified water, the column was washed with purified water and trehalose was eluted with 20% ethanol. The eluate was analyzed by the method described in Example 1 and no sugar was found except trehalose. The eluate was further concentrated to give about 243 mg of white powder.

### [Example 3]

### (Process 1)

In a test tube with a screw cap, 1 ml of 880 mM sucrose aqueous solution, 1 ml of 880 mM potassium phosphate buffer (pH 7.0), 0.68 ml of 500 mM HEPES buffer (pH 7.0), 0.18 ml of 18 unit/ml aqueous solution of sucrose phosphorylase prepared by Preparation example 2 and 0.14 ml of purified water were mixed and incubated at 35°C for 20 hrs., then the enzyme was inactivated by heating at 100°C for 10 min. Determination of α-glucose 1-phosphate in the reaction mixture was carried out by the following method and 220 mM of α-glucose 1-phosphate was found. The fructose concentration in the reaction mixture was determined in a manner similar to that of trehalose shown in Example 1 and 225 mM of fructose was found, indicating the yield of 75 and 77 mol% for α-glucose 1-phosphate and fructose from sucrose, respectively.

The concentration of α-glucose 1-phosphate was determined by the following method, that is, in a portion of 2.5 ml from 100 ml of 47 mM potassium phosphate buffer (pH 7.0) containing 17.1 mg of EDTA 2Na, 100 µl of 14.8 mM NADP⁺ aqueous solution, 100 µl of 26 mM magnesium chloride aqueous solution, 100 µl of 1.34 mM glucose 1,6-diphosphate aqueous solution, 50 µl of 31 unit/ml of phosphoglucomutase aqueous solution and 50 µl of 35 unit/ml of glucose-6-phosphate dehydrogenase aqueous solution, and 100 µl of the test sample were mixed and incubated at 30°C for 30 min. The produced NADPH was spectrophotometrically determined with the absorbance at 340 nm to give the amount of α-glucose 1-phosphate.

### (Process 2)

A mixture of 2.75 ml of the above reaction (process 1) ended by the inactivation of the enzyme containing α-glucose 1-phosphate and fructose, 0.06 ml of 1M magnesium sulfate and 0.19 ml of 16.2 unit/ml of purified glucose isomerase obtained in Preparation example 3 were mixed and incubated at 60°C for 5 hrs. in a screw capped test tube. The reaction mixture was heated and the enzyme was inactivated. In the reaction mixture, 101 mM of glucose was found by determination with a glucose determination kit (glucose CII-test Wako, Wako Pure Chemical Ind. Ltd.) with a yield of 49 mol% of glucose produced from fructose.

### (Process 3)

A mixture of α-glucose 1-phosphate produced by process 1 and the reaction product of process 2 containing glucose was heated to inactivate the enzyme. A mixture of 2.71 ml of the above mentioned treated aqueous solution and 0.29 ml of 10.8 unit/ml of trehalose phosphorylase prepared by Preparation example 1 was incubated at 30°C for 16 hrs. in a test tube with screw cap. The enzyme in the reaction mixture was inactivated by heating and the amount of trehalose in the treated reaction mixture was determined in a manner similar to that of Example 1 and 62 mM of trehalose was found. The yield of trehalose from glucose was 61 mol%. The yield of trehalose from process 1 through 3 was about 26 mol%.

### [Example 4]

In a test tube with screw cap, 0.9 ml of 1M sucrose aqueous solution, 0.12 ml of 500 mM potassium phosphate buffer (pH 7.0), 0.4 ml of 750 mM HEPES buffer (pH 7.0), 0.06 ml of 1M magnesium sulfate aqueous solution, 0.29 ml of 10.8 unit/ml of trehalose phosphorylase aqueous solution prepared by Preparation example 1, 0.18 ml of 18 unit/ml of sucrose phosphorylase aqueous solution prepared by Preparation example 2, 0.4 ml of 3.8 unit/ml of glucose isomerase aqueous solution prepared by the addition of sodium sulfate in Preparation example 3, and 0.65 ml of purified water were placed, mixed and incubated at 30°C for 23 hrs. with or without the replacement of the air in the test tube with argon gas. The enzyme in the reaction mixtures was inactivated by heat treatment, the content of trehalose was determined by the method described in Example 1 and 109 and 89 mM trehalose was found, respectively.

## Claims

1. A process for the production of trehalose characterized by the production of trehalose by a reaction of sucrose with an inorganic phosphoric acid and/or a salt thereof, sucrose phosphorylase, glucose isomerase and trehalose phosphorylase, followed by isolation of trehalose.

2. A process for the production of trehalose characterized by the production of trehalose by a reaction of a mixture of sucrose, an inorganic phosphoric acid and/or a salt thereof, sucrose phosphorylase, glucose isomerase and trehalose phosphorylase, followed by isolation of trehalose.

3. A process for the production of trehalose consisting of the following steps:
(Process 1) A process for the production of α-glucose 1-phosphate and fructose by a reaction of sucrose with an inorganic phosphoric acid and/or a salt thereof catalyzed by sucrose phosphorylase.
(Process 2) A process for the production of glucose by a reaction of fructose prepared by the Process 1 with glucose isomerase.
(Process 3) A process for the production of trehalose and an inorganic phosphoric acid by a reaction of α-glucose 1-phosphate prepared by the Process 1, glucose prepared by the Process 2 with trehalose phosphorylase, followed by an isolation of trehalose.

4. A process for the production of trehalose according to one of claims 1-3, wherein the amount of inorganic phosphoric acid and/or salts thereof used is in a range of from 0.01 millimole to one mole for one mole of sucrose.

5. A process for the production of trehalose according to one of claims 1-4, wherein each amount of sucrose phosphorylase, glucose isomerase and trehalose phosphorylase used is in a range of from 0.01 or more, preferably 0.1-100 units for one gram of sucrose.

6. A process for the production of trehalose according to one of claims 1-5, characterized by the production thereof under anaerobic conditions.

7. A process for the production of trehalose characterized by the addition of an invertase to the reaction mixture prepared according to one of claims 1-6 to convert unaltered sucrose into glucose and fructose followed by the isolation of trehalose.
